(19) Europäisches Patentamt
European Patent Office
Office européen des brevets

(11) Veröffentlichungsnummer : **0 182 111**
**B1**

# (12) EUROPÄISCHE PATENTSCHRIFT

(45) Veröffentlichungstag der Patentschrift : **20.09.89**

(21) Anmeldenummer : **85113286.0**

(22) Anmeldetag : **19.10.85**

(51) Int. Cl.[4] : **C 07 B 39/00**, C 07 D241/52, C 07 D241/16, C 07 D213/84, C 07 D333/28, C 07 D333/38, C 07 D333/62

## (54) Verfahren zur Herstellung von perhalogenierten Verbindungen.

(30) Priorität : **20.10.84 DE 3438567**
**05.02.85 DE 3503774**
**01.04.85 DE 3511926**

(43) Veröffentlichungstag der Anmeldung : **28.05.86 Patentblatt 86/22**

(45) Bekanntmachung des Hinweises auf die Patenterteilung : **20.09.89 Patentblatt 89/38**

(84) Benannte Vertragsstaaten :
**CH DE FR GB IT LI**

(56) Entgegenhaltungen :
**DE-B- 1 285 467**
**DE-B- 1 468 465**
**GB-A- 613 109**
**GB-A- 1 301 557**
**US-A- 2 442 473**
**US-A- 2 492 624**
**US-A- 2 608 591**
**US-A- 3 325 503**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Neumann, Peter, Dr.**
**Franz-Schubert-Strasse 1**
**D-6908 Wiesloch (DE)**
Erfinder : **Bender, Herbert, Dr.**
**Koenigsberger Strasse 5**
**D-6737 Boehl-Iggelheim (DE)**

**EP 0 182 111 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Aus der Literatur sind mehrere Verfahren zur Herstellung von Hexachlorchinoxalin bekannt. Burton und Mitarbeiter beschreiben in J.C.S. C 1968, 1268-73 die Cyclisierung von Tetrachlor-o-phenylendiamin zum Hexachlorchinoxalin. Die schlechte Verfügbarkeit von Tetrachlorphenylendiamin sowie die ungenügende Ausbeute bei der Cyclisierung erlauben keine Herstellung im industriellen Maßstab.

Allison und Mitarbeiter (J. Fluorine Chem. 1971, 59-67) gehen zwar vom leicht zugänglichen Dichlorchinoxalin aus, aus dem die Hexachlorverbindung durch Chlorieren mit $PCl_5$ bei 300 °C hergestellt wird. Dieses sehr korrosive Gemisch stellt jedoch an die Chlorierungsapparatur hohe Anforderungen. Zur technischen Herstellung ist dieses Verfahren somit nur schlecht geeignet.

In Chem. Ber. 107, 558-565, beschreibt A. Roedig die Synthese von Hexachlorchinoxalin durch Pyrolyse von 2,3-Diazido-hexachlorbicyclo[4,2,0]octa-1,5,7-trien. Aus naheliegenden Gründen kann dieser Syntheseweg nicht zur industriellen Produktion benutzt werden.

Weiterhin ist aus der DE-OS 1 670 729 die Herstellung von Hexachlorchinoxalin durch Erhitzen von Pentachlor-phenylimino-chlorcarbonylcyanid bekannt. Wegen der schlechten Zugänglichkeit der Ausgangsverbindung erlaubt dieses Verfahren keine kostengünstige technische Herstellung von Hexachlorchinoxalin.

Aus der US-A-3 532 701 ist ein Verfahren zur Herstellung perchlorierter Pyridine und Cyanpyridine durch Chlorierung von mono- und dicyansubstituierten Cyclobutanen und Cyclobutenen bekannt. Da nach diesem Verfahren nur schwer trennbare Gemische cyansubstituierter Pyridine erhalten werden, ist nach diesem Verfahren keine industrielle Produktion möglich.

In der US-A-3 420 833 wird die Herstellung von perchlorierten Cyanpyridinen durch Chlorierung der entsprechenden Cyanpyridine in einem leeren Rohr ohne Katalysator beschrieben. Die Ausbeuten liegen bei diesem Verfahren unter 80 %. Die starke Verdünnung der Ausgangsmaterialien mit chlorierten organischen Lösungsmitteln wie Tetrachlorkohlenstoff und Tetrachlorethylen steht einer industriellen Durchführung ebenso entgegen wie der geringe Durchsatz von 1 bis 2 $g/cm^2 \cdot h$.

In der US-A-3 325 503 wird die Chlorierung von Cyanpyridinen an einem Festbettkatalysator in der Gasphase beschrieben. Die Reaktion erfolgt bei 200 bis 500 °C in Gegenwart von Katalysatoren, wie Aluminiumoxid, Siliciumdioxid, Kaolin oder ähnlichen Tonen (Clays), Eisenoxiden, vorzugsweise in Gegenwart von aktiviertem Kohlenstoff. Vorteilhafterweise wird der Katalysator mit Stoffen, wie Bariumchlorid modifiziert, wobei in diesem Zusammenhang Kohle besonders hervorgehoben wird (Sp. 2, Z. 15/21).

Die Ausbeuten liegen nach den Angaben in den Beispielen zwischen 35 und 60 %, der Durchsatz zwischen 1 und 2 $g/cm^2 \cdot h$. Ein analoges Verfahren wird in der US-A-3 652 637 beschrieben, bei dem die Ausgangsnitrile durch Ammonoxidation aus den entsprechenden Methylverbindungen unmittelbar vor der Chlorierungsstufe hergestellt werden. Ausbeute und Durchsatz sind hier noch ungünstiger. Die Aufarbeitung und die Isolierung der gewünschten Verfahrensprodukte ist nur im Labormaßstab möglich.

In der US-A-2 442 473 wird die Herstellung von Tetrachlorpyrazinen beschrieben. Nach den Angaben in Sp. 2, Z. 51 bis Sp. 3, Z. 9 kann die Chlorierung nicht in einer Stufe erfolgen, da sonst die Reaktion sehr heftig und unkontrolliert abläuft. Aus diesem Grunde erfolgt die Umsetzung a) in Gegenwart von Wasserdampf und b) wird das Halogen (Chlor) so zudosiert, daß das Verhältnis Halogen : Pyrazin nicht größer als 1,5 : 1 Mol ist. Im Falle der Herstellung von Perchlorpyrazin wird das Reaktionsprodukt in gleicher Weise erneut chloriert. Das dabei gebildete Tetrachlorpyrazin wird erst dann abgetrennt, wenn bestimmte Konzentrationen daran im Reaktionsgemisch erreicht sind (Sp. 3, Z. 20/28).

Die bekannten Verfahren zur Herstellung von Tetrachlorthiophen und 2-Cyanotrichlorthiophen beruhen auf der Cyclisierung chlorierter Kohlenwasserstoffe mit elementarem Schwefel bzw. Schwefelwasserstoff. Alle diese Verfahren arbeiten bei hohen Drucken und benötigen als Ausgangsstoffe teilweise toxikologisch bedenkliche chlorierte Kohlenwasserstoffe. Die Ausbeuten an den gewünschten Verfahrensprodukten ist in den meisten Fällen nur sehr unbefriedigend. In der GB-A-1 069 943 wird, ausgehend von Tetrachlorethylen und Schwefel, bei 250 °C Reaktionstemperatur nur 13 % Tetrachlorthiophen isoliert. Nach Khim. Geterotskil. Soedin. 1980, S. 310-311 erhält man aus Tetrachlorethylen und Schwefelwasserstoff Tetrachlorthiophen in Ausbeuten von 74 %, jedoch erfordert die Reaktionstemperatur von 450 °C sehr hohe Drücke. Als weitere Ausgangsstoffe wurden z. B. Hexachlorbutadien, Octachlorbutan, Trichlorethylen verwendet. In der PL-PS 74 672 wird die Chlorierung von Thiophen mit Chlor in Gegenwart von Schwefel, Schwefeldichlorid und Dischwefeldichlorid bei 100 °C unter Druck (30 bar) beschrieben. Als Reaktionsprodukt wird jedoch kein Tetrachlorthiophen erhalten. Durch Weiterchlorierung tritt Bildung von Octachlorthiolan ein.

Aus Ed. Sci. 21 (11), 811-17 (1966) ist bekannt, daß Dichlorpyrazin durch photochemische Chlorierung in Tetrachlorpyrazin überführt werden kann. Aus wirtschaftlichen Gründen ist dieses Verfahren nicht für eine industrielle Produktion geeignet.

In der GB-A-1 163 582 ist die Chlorierung von Chlorpyrazin und Piperazin mit $PCl_5$ zum Tetrachlorpyrazin beschrieben. Der große $PCl_5$-Überschuß, die langen Reaktionszeiten sowie die sehr aufwendige Aufarbeitung erlauben keine wirtschaftliche Herstellung.

In der DE-A-1 911 023 wird die Chlorierung von Piperazin sowie von N-Methyl- und N,N'-Dimethyl-

piperazin in der Gasphase zum Tetrachlorpyrazin beschrieben. Bei diesem Verfahren muß nach den Angaben in den Beispielen ein großer Chlorüberschuß angewendet werden: je Mol Tetrachlorpyrazin mehr als 50 Mol Chlor. Die Ausbeuten liegen nur bei 26 bzw. 38 %.

Aufgabe der vorliegenden Erfindung war es, ein Verfahren zur Herstellung von perhalogenierten Thiophenen, Benzthiophenen, Pyrazinen, Cyanpyridinen und Chinoxalinen bereitzustellen, das die Nachteile der Verfahren des Standes der Technik nicht aufweist und das die perhalogenierten Verbindungen in hoher Ausbeute bei gleichzeitig hoher Reinheit liefert.

Es wurde gefunden, daß man perhalogenierte Verbindungen aus der Reihe der Thiophene, Benzthiophene, Pyrazine, Cyanpyridine oder Chinoxaline erhält, wenn man die Verbindungen im Wirbelbett in Gegenwart von Aktivkohle, Koks oder Gemischen davon als Halogenierungskatalysatoren mit Chlor, Brom oder Gemischen davon bei Temperaturen von 300 bis 800 °C umsetzt.

Nach dem Verfahren erhält man die perhalogenierten Verbindungen in sehr guten Ausbeuten, hohen Reinheiten und hohen Raum-Zeit-Ausbeuten. So tritt z. B. bei dem erfindungsgemäßen Verfahren bei den Thiophenen weder Überhalogenierung zu Perhalogenothiolanen noch Spaltung des Thiophenrings zu acyclischen Halogenierungsprodukten auf. Die Ausbeuten liegen bei über 90 % der Theorie, die Reinheit der Verfahrensprodukte oberhalb 90 %. Die Halogenierung erfolgt unter sehr günstigen Raum-Zeit-Ausbeuten von über 5 $g/cm^2$ h. Im Falle der Chlorierung von Pyrazinen war es im Vergleich zur DE-A-1 911 023 überraschend, daß nach dem erfindungsgemäßen Verfahren ein nur geringer Chlorüberschuß zur erwünschten Perchlorierung ausreicht. Die erzielten Raum-Zeit-Ausbeuten von über 4 $g/cm^2$ waren aufgrund der Angaben in der DE-A (< 0,5 $g/m^2$) nicht zu erwarten, desgleichen nicht die Reinheiten von über 99 % bei quantitativer Umsetzung.

Die perhalogenierten Verfahrensprodukte sind wertvolle Vorprodukte für Farbstoffe, Pflanzenschutzmittel sowie Additive für Kunststoffe.

Das Verfahren gemäß der Erfindung kann man in einem einfachen oder unterteilten, offenen oder geschlossenen Wirbelschichtsystem mit oder ohne Fließbettzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 2, S. 480 ff (1973) verwiesen.

Die Reaktion kann z. B. wie folgt durchgeführt werden:

Man leitet kontinuierlich Chlor oder Brom sowie die dampfförmigen zu halogenierenden Verbindungen und gegebenenfalls Inertgas bei der Reaktionstemperatur durch den Reaktor. Die Gasgemische können so durch den im Reaktor befindlichen Katalysator geleitet werden, daß der fluide Zustand einer Wirbelschicht aufrechterhalten wird. Das Wirbelschichtverfahren kann dabei mit aber auch ohne Inertgas durchgeführt werden, wobei im letzteren Fall die Ausgangsstoffe selbst als Wirbelgas dienen.

Zweckmäßigerweise werden die Ausgangsstoffe in der Gasphase in Gegenwart von unter den Reaktionsbedingungen inertem Gas als Verdünnungsmittel umgesetzt. Als unter den Reaktionsbedingungen inerte Gase kommen Edelgase, Stickstoff, Kohlendioxid, Kohlenmonoxid, Chlorwasserstoff oder Gemische davon, vorzugsweise Stickstoff, in Betracht. Die Ausgangsstoffe Chinoxalin und Halogen können, gegebenenfalls mit Inertgas verdünnt, vor der Reaktionszone vereinigt werden. Man kann auch eine oder beide Ausgangsverbindungen direkt in den Katalysator eindosieren. Vorzugsweise werden Chinoxalin und Halogen direkt unterhalb der Reaktionszone eindosiert.

Die Reaktionstemperaturen liegen bei 300 bis 800 °C, vorzugsweise bei 320 bis 700 °C.

Als Katalysatoren kommen für das Verfahren gemäß der Erfindung Aktivkohle und Koks in Betracht (spez. Oberfläche von 100 bis über 1 200 $m^2/g$ bzw. unter 1 $m^2/g$ bis 100 $m^2/g$).

Die genannten Katalysatoren können alleine oder als Gemisch in jeder beliebigen Zusammensetzung untereinander angewendet werden.

Bevorzugt ist eine Strömungsgeschwindigkeit der Gase von 0,01 bis 10, vorzugsweise von 0,05 bis 1 $m \cdot s^{-1}$. Es können auch wesentlich niedrigere und auch höhere Gasgeschwindigkeiten benutzt werden, doch bringt dies bei dem erfindungsgemäßen Verfahren keinen Vorteil. Der Durchsatz liegt bei der Umsetzung zwischen 1 und 200, vorteilhafterweise zwischen 5 und 100 g Chinoxalin pro $cm^2$ Reaktorfläche pro Stunde.

Die halogenierten Produkte werden in üblicher Weise aus dem Reaktionsgemisch abgetrennt. Vorteilhafterweise wird das Reaktionsgemisch durch Quenchen mit Wasser abgekühlt und aus der erhaltenen Suspension das Verfahrensprodukt durch Absaugen isoliert. Die so erhaltenen perhalogenierten Verbindungen haben Reingehalte von 92 bis 99 % (nach gaschromatographischer Analyse). Die erzielten Ausbeuten liegen zwischen 90 und 99 % der Theorie.

Als Ausgangsstoffe kommen z. B. in Betracht: Chinoxalin, 2,3-Dichlor- und 2,3-Dibromchinoxalin, Monocyanpyridine, Dicyanpyridine, Tricyanpyridin, Thiophen, Benzthiophen, 2-Cyano- und 3-Cyanothiophen, 2-Chlor- und 3-Chlorthiophen, 2-Brom- und 3-Brom-thiophen, 2,3-Dicyano-, 2,4-Dicyano- und 2,5-Dicyanothiophen, 3,4-Dicyanothiophen, Chlor-cyano-thiophen, 2-, 3-, 4- und 7-Cyano-benzthiophen sowie Chlorcyano-benzthiophene, Pyrazin, Chlorpyrazin, 2,3- und 2,6-Dichlorpyrazin, Brom- und Dibrompyrazin, 2-Cyanopyrazin, 2,3- und 2,5- und 2,6-Dicyanopyrazin, 3-Chlor-2-cyano-pyrazin, 5-Chlor-2-cyanopyrazin und 6-Chlor-2-cyanopyrazin.

Die Ausgangsstoffe können beim erfindungsgemäßen Verfahren in stöchiometrischer Menge oder im Überschuß jeder Komponente zur anderen, vorteilhafterweise mit einem Halogenüberschuß, angewendet werden. Zur Halogenierung wendet man in der Regel die 1,0 bis 2,5-fache, vorzugsweise die 1,05 bis 2,0-

fache der stöchiometrisch erforderlichen Menge an Chlor und/oder Brom an.

Bei den monocyansubstituierten Pyridinen verwendet man zweckmäßig ein Verhältnis von 4 bis 10 Mol Chlor und/oder Brom, bevorzugt 4 bis 7 Mol je Mol Monocyanpyridin. Bei den dicyan- und tricyansubstituierten Pyridinen wählt man zweckmäßig ein Verhältnis von 3, bzw. 2 bis 8 Mol Chlor und/oder Brom, bevorzugt 3 bis 6 Mol je Mol Ausgangsverbindung. Bei der Verwendung mono- oder dihalogenierter Ausgangsverbindungen reduziert sich die erforderliche Halogenmenge entsprechend dem Halogenierungsgrad.

Die Reaktion kann durch folgende Reaktionsgleichung wiedergegeben werden :

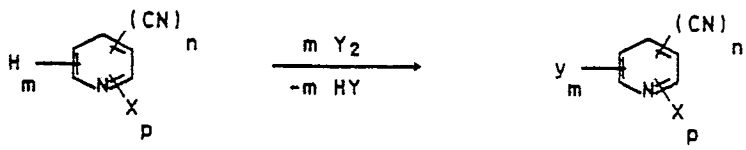


X = Cl oder Br
Y = Cl oder Br
m = 1, 2, 3, oder 4
n = 1, 2, 3 oder 4
p = 0, 1 oder 2 wobei
(m + n + p) = 5

Vorteilhafterweise wendet man beim Chinoxalin einen Überschuß an Halogen an, z. B. 6,5 bis 18, vorzugsweise 6,5 bis 14 Mol Chlor je Mol Chinoxalin. Bei 2,3-Dichlor- oder 2,3-Dibromchinoxalin werden zweckmäßigerweise 4,3 bis 12, vorzugsweise 4,5 bis 8 Mol Chlor oder Brom je Mol zu halogenierender Verbindung angewendet.

Die Reaktion kann hier durch folgende Reaktionsgleichungen wiedergegeben werden :

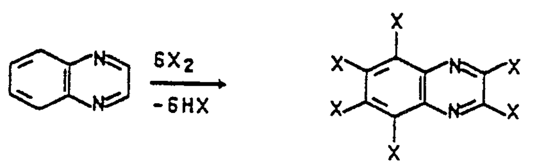


X = Cl oder Br

(III) (IV)

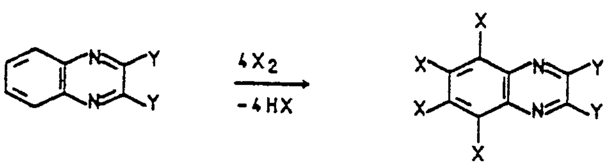


Y = Cl oder Br
X = Cl oder Br

(II) (I)

Nach dem erfindungsgemäßen Verfahren sind auch perhalogenierte Chinoxaline mit Chlor und Brom im gleichen Molekül zugänglich, beispielsweise aus 2,3-Dibromchinoxalin (Y = Br, X = Cl in Formel (I)), 2,3-Dibrom-5,6,7,8-tetrachlorchinoxalin.

Bei Thiophen verwendet man zweckmäßig ein Verhältnis von 4 bis 8 Mol Chor, bevorzugt 4 bis 6 Mol je Mol Thiophen, bei cyano- und halogensubstituierten Thiophenen zweckmäßig ein Verhältnis von 3 bis 8 Mol Chlor, vorzugsweise von 3 bis 6 Mol Chlor je Mol Ausgangsverbindung.

Die Umsetzungen können hier durch folgende allgemeine Reaktionsgleichungen wiedergegeben werden.

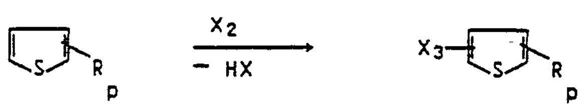


R = H, 2—CN, 3—CN, Cl, Br

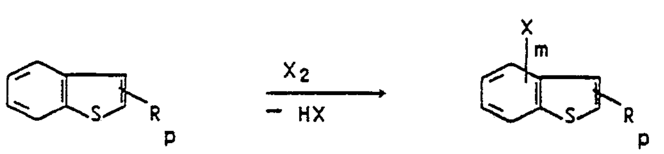


m + p = 6

Bei Pyrazin verwendet man zweckmäßig ein Verhältnis von 2 bis 8 Mol Halogen, bevorzugt 4 bis 6 Mol je Mol Pyrazin, bei mono- und dicyanosubstituierten Pyrazinen zweckmäßig ein Verhältnis von 2 bzw. 3

bis 7 Mol, bevorzugt 3 bis 6 Mol Halogen je Mol Ausgangsverbindung. Bei Verwendung mono- oder dihalogenierter Pyrazine reduziert sich gemäß dem Halogenierungsgrad die notwendige Halogenmenge.

Die Umsetzungen können durch folgende allgemeine Reaktionsgleichung wiedergegeben werden :

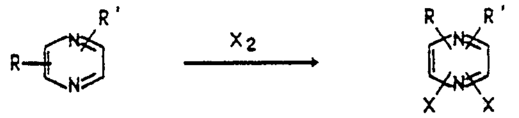


R, R' : H, CN, Halogen

X : Cl, Br

Bei der Herstellung hydrolyseempfindlicher Pyrazine, z. B. 2,3-Dicyano-5,6-dichlorpyrazin wird vorteilhafterweise ein inertes Lösungsmittel als Quenchflüssigkeit verwendet.

Der Aufarbeitungsprozeß kann kontinuierlich durchgeführt werden. Die so hergestellten perhalogenierten Pyrazine werden in einer Reinheit von 95 bis über 99 % (nach gaschromatographischer Analyse) erhalten. Die erzielten Ausbeuten liegen über 95 % der Theorie.

3-Cyanotrichlorthiophen ist neu and wurde erstmals durch Gasphasenchlorierung von 3-Cyanothiophen dargestellt.

Das Verfahren soll durch die folgenden Beispiele zusätzlich erläutert werden.

Beispiel 1

In einem Stickstoffstrom von 600 l/h wurden je h 350 g (= 1,7 Mol) 2,3-Dichlorchinoxalin bei 350 °C verdampft. Das Chinoxalin-Stickstoffgemisch wurde in einem Wirbelbettreaktor (60 mm lichte Weite ; 1 000 mm Höhe ; Füllung 240 g Aktivkohle mit einer spezifischen Oberfläche von 1 000 $m^2$/g und einer Körnung von 0,1 bis 0,4 mm) bei 640 °C eingeleitet. Gleichzeitig wurden kontinuierlich je h 300 l Chlor direkt in das Wirbelbett eingeleitet. Am Ende des Reaktors wurde der abziehende Gasstrom mit Wasser gequencht (25 °C). Dabei wurden je h 555 g (= 98 % der Theorie) Hexachlorchinoxalin mit einem Reingehalt von 95 % isoliert. Die gleiche Ausbeute wurde auch noch nach 1 000 Betriebsstunden erhalten.

Beispiel 2

In der im Beispiel 1 beschriebenen Apparatur wurden je h 600 g (3 Mol) Dichlorchinoxalin mit 600 l N2 pro Stunde verdampft und bei 600 °C kontinuierlich mit 340 l Chlor pro Stunde zur Reaktion gebracht. Das Wirbelbett enthielt 300 g Aktivkohle, welche mit $BaCl_2$ dotiert war. Es wurden 929 g (92 % der Theorie) Hexachlorchinoxalin isoliert.

Beispiel 3

Analog Beispiel 1 wurden 195 g (1,5 Mol) Chinoxalin mit 100 l Stickstoff pro Stunde verdampft und bei 580 °C mit 400 l Chlor pro Stunde in einem Wirbelbett mit Petrokoks als Katalysator zur Reaktion gebracht. Es wurden je Stunde 485 g Hexachlorchinolin (96 % der Theorie) erhalten.

Beispiel 4

80 g (0,77 Mol) Picolinnitril wurden pro Stunde bei 260 °C in einem Stickstoffstrom (80 l/h) verdampft. Das Picolinnitril-Stickstoff-Gemisch wurde in einem Wirbelbettreaktor mit 25 mm lichter Weite bei 550 °C eingeleitet. Kontinuierlich wurden 315 g (4,4 Mol Chlor pro Stunde direkt ins Wirbelbett mit einer Füllung aus 15 ml Aktivkohle (spezifische Oberfläche : 1 000 $m^2$/g) mit einer Körnung von 0,1 bis 0,4 mm eingeleitet. Aus dem Gasstrom wurden am Ende des Reaktors mit einem Wasserquench bei 25 °C 185 g (98 % der Theorie) Tetrachlor-2-cyanpyridin stündlich isoliert. Das Rohprodukt enthielt nur 4 % Pentachlorpyridin und war für die meisten Anwendungszwecke ohne weitere Reinigungsoperation verwendbar. Die vorgenannten Ergebnisse wurden auch nach 1 000 Betriebsstunden erhalten.

Beispiel 5

250 g (2,4 Mol) Nicotinsäurenitril wurden pro Stunde bei 240 °C mit 650 l Stickstoff pro Stunde verdampft. Das Gemisch wurde in einem Wirbelbettreaktor mit 60 mm lichter Weite bei 590 °C eingeleitet. 825 g/h (11,6 Mol) Chlor wurden direkt unter das Wirbelbett, bestehend aus 150 ml Petrolkoks, eingeleitet. 563 g (97 % der Theorie) Tetrachlornicotinsäurenitril mit einer Reinheit von 98 % (ca. 1 % Pentachlorpyridin) wurden erhalten.

Beispiel 6

In einer in Beispiel 4 beschriebenen Apparatur wurden stündlich kontinuierlich 104 g (1 Mol) 4-Cyanpyridin mit 40 l Stickstoff verdampft und bei 575 °C mit 570 g (8 Mol) Chlor pro Stunde in einem

Wirbelbett mit Aktivkohle als Katalysator, welcher mit $PdCl_2$ dotiert war, zur Reaktion gebracht. Es wurden 230 g (95 % der Theorie) Tetrachlor-4-cyanpyridin erhalten.

Beispiel 7

42 g (0,3 Mol) Thiophen wurden pro Stunde bei 300 °C in einem Stickstoffstrom verdampft. Das Thiophen-Stickstoff-Gemisch wurde in einen Wirbelbettreaktor mit 25 mm lichter Weite bei 600 °C eingeleitet. Kontinuierlich wurden 78 l Chlor pro Stunde direkt ins Wirbelbett mit einer Füllung aus 20 ml Aktivkohle mit einer Oberfläche von 1 200 $m^2/g$ eingeleitet. Aus dem Gasstrom wurden mit einem Wasserquench 100 g pro Stunde (90 % d. Theorie) Tetrachlorthiophen mit einer Reinheit von 90 % (laut gaschromatographischer Analyse) isoliert. Auch nach mehrwöchiger Fahrweise wurden die gleichen Ergebnisse erzielt.

Beispiel 8

In der unter Beispiel 7 beschriebenen Apparatur wurden bei 625 °C 37 g (0,3 Mol) 2-Cyanothiophen und 30 l Chlor pro Stunde an einem Acetylenkokskatalysator mit 90 l Stickstoff pro Stunde als Trägergas umgesetzt. Es wurden 70 g (98 % der Theorie) einer farblosen Verbindung isoliert, die zu 93 % (laut Gaschromatogramm) aus 2-Cyano-trichlorthiophen besteht.

Beispiel 9

In der unter Beispiel 7 beschriebenen Apparatur wird anstatt Thiophen 59 g (0,5 Mol) Chlorthiophen eingesetzt, die Chlormenge betrug 45 l/h. Unter sonst gleichen Bedingungen wurden die gleichen Ergebnisse wie unter 7 erzielt.

Beispiel 10

Analog Beispiel 7 wurden 30 g Benzothiophen mit 50 l Chlor und 70 l Stickstoff pro Stunde bei 590 °C umgesetzt. Es wurden stündlich 70 g (92 %) Hexachlorbenzothiophen isoliert.

Beispiel 11

160 g (2 Mol) Pyrazin wurden pro Stunde bei 200 °C in einen Stickstoffstrom (500 l/h) verdampft. Das Pyrazin-Stickstoff-Gemisch wurde in einem Wirbelbettreaktor mit 60 mm lichter Weite bei 600 °C eingeleitet. Kontinuierlich wurden 200 l Chlor pro Stunde direkt ins Wirbelbett mit einer Füllung aus 150 ml Petrolkoks mit einer Körnung von 0,1 bis 0,4 mm eingeleitet. Aus dem Gasstrom wurden am Ende des Reaktors mit einem Wasserquench bei 20 °C 430 g (99 % der Theorie) Tetrachlorpyrazin stündlich isoliert. Reinheit: 99 % laut gaschromatographischer Analyse. Die gleichen Ergebnisse wurden auch nach 1 000 Betriebsstunden erhalten.

Beispiel 12

130 g (1 Mol) 3,4-Dicyanopyrazin wurden bei 250 °C in einem Stickstoffstrom verdampft und in die unter Beispiel 7 beschriebene Apparatur bei 580 °C eingeleitet. In das Wirbelbett, bestehend aus 200 g Aktivkohle (spez. Oberfläche: 1 200 $m^2/g$), wurden gleichzeitig 90 l/h Chlor geleitet. Mittels eines Lösungsmittelquenches (Toluol oder Dichlormethan) wurden 190 g Dichlor-dicyanopyrazin erhalten, das in allen spektroskopischen Eigenschaften identisch mit nach bekannten Literaturverfahren hergestellter Ware war.

Beispiel 13

In der unter Beispiel 7 beschriebenen Apparatur wurden bei 625 °C 229 g (2 Mol) Chlorpyrazin und 200 l Chlor pro Stunde in einem Wirbelbettreaktor, mit $PdCl_2$ dotierter Aktivkohle, zur Reaktion gebracht. Als Wirbelgas wurden 400 l/h Stickstoff verwendet. Es wurden 427 g (98 %) Tetrachlorpyrazin isoliert.

## Patentansprüche

1. Verfahren zur Herstellung von perhalogenierten Verbindungen aus der Reihe der Thiophene, Benzthiophene, Pyrazine, Cyanpyridine oder Chinoxaline, dadurch gekennzeichnet, daß man die Verbindungen im Wirbelbett in Gegenwart von Aktivkohle, Koks oder Gemischen davon als Halogenierungskatalysatoren mit Chlor, Brom oder Gemischen davon bei Temperaturen von 300 bis 800 °C umsetzt.

2. Verfahren gemäß Anspruch 1, dadurch gekennzeichnet, daß die Halogenierung bei 320 bis 700 °C erfolgt.

3. Verfahren gemäß Anspruch 1 oder 2, dadurch gekennzeichnet, daß Chlor und/oder Brom in mindestens der stöchiometrischen Menge, mit oder ohne Verdünnungsmittel angewendet werden.

4. Verfahren gemäß Anspruch 1 oder 2, dadurch gekennzeichnet, daß man zur Halogenierung die 1,0- bis 2,5-fache, vorzugsweise 1,05- bis 2,0-fache der stöchiometrisch erforderlichen Menge an Chlor und/oder Brom anwendet.

## Claims

1. A process for the preparation of a perhalogenated compound from the series consisting of the thiophenes, benzothiophenes, pyrazines, cyanopyridines or quinoxalines, wherein the compound is reacted with chlorine, bromine or a mixture of these at from 300 to 800 °C in the presence of active carbon, coke or a mixture of these as a fluidizedbed halogenation catalyst.

2. A process as claimed in claim 1, wherein the halogenation is carried out at from 320 to 700 °C.

3. A process as claimed in claim 1 or 2, wherein chlorine bromine and/or is used in not less than the stoichiometric amount with or without a diluent.

4. A process as claimed in claim 1 or 2, wherein the halogenation is carried out using from 1.0 to 2.5, preferably from 1.05 to 2.0, times the stoichiometric amount of chlorine and/or bromine.

## Revendications

1. Procédé de préparation de composés perhalogénés de la série des thiophènes, benzothiophènes, pyrazines, cyanopyridines ou quinoxalines, caractérisé en ce que l'on fait réagir les composés en lit fluidisé, en présence de charbon actif, de coke ou de mélanges de ces substances comme catalyseurs d'halogénation, avec du chlore, du brome ou des mélanges de ceux-ci, à des températures de 300 à 800 °C.

2. Procédé suivant la revendication 1, caractérisé en ce que l'halogénation a lieu à une température de 320 à 700 °C.

3. Procédé suivant la revendication 1 ou 2, caractérisé en ce que le chlore et/ou le brome sont utilisés au moins en la quantité stœchiométrique, avec ou sans diluant.

4. Procédé suivant la revendication 1 ou 2, caractérisé en ce que l'on utilise pour l'halogénation la quantité 1,0 à 2,5 fois, de préférence 1,05 à 2,0 fois stœchiométriquement nécessaire de chlore et/ou de brome.